# EUROPEAN PATENT APPLICATION

(11) **EP 4 275 627 A1**
(43) Date of publication of application: **15.11.2023**
(21) Application number: 22173203.5
(22) Date of filing: 13.05.2022
(51) Int. Cl.: A61B 17/3213, A61B 17/322

(54) **DISPOSABLE SURGICAL BLADE AND INSTRUMENTS FOR HARVESTING TISSUE**

(71) Applicant: De Greef Alexander BV, 1840 Londerzeel (BE)
(72) Inventor: De Greef, Alexander, 1840 Londerzeel (BE)
(74) Representative: Clerinx, Peter Albert I.

(57) **Abstract**

The present invention relates to a disposable surgical blade (1) for harvesting tissue material, comprising a U-shaped plate (2), to a sterilizable surgical blade holder (20) for holding the blade (1), to a sterilizable surgical knife handle (30) for rotatably holding the blade holder (20), and to a kit-of-parts comprising the aforementioned devices. The U-shaped plate comprises a base part (3) and two lateral parts (4) that are connected by two corresponding joint regions (5) to the base part. The base part (3) has a sharpened frontal edge (6) spanning the entire width (W) of the base part. Each lateral part (4) has a sharpened frontal edge (7) that extends over at least a section of a frontal edge of the lateral part, such that the sharpened frontal edge (7) of each lateral part abuts against and/or continuously joins with the sharpened frontal edge (6) of the base part.

## Description

### Technical field of the invention

The present invention relates to the field of surgical instruments, e.g. for periodontal surgery, and, more specifically, the invention relates to a disposable surgical blade for harvesting tissue material, a sterilizable surgical blade holder for holding the blade, a sterilizable surgical knife handle for rotatably holding the blade holder, and a kit-of-parts comprising the aforementioned devices.

### Technical background of the invention

It is known in the art to harvest a gum tissue graft in the treatment of various mucogingival problems and/or in periodontal plastic surgery. In such grafting procedure, a small layer of tissue may be removed from the palate, and then relocated to the target site, e.g. a site of receding gums, where it serves to protect the exposed root and/or increases the thickness of thinning gum tissue. The living tissue graft at the target site, and the remaining tissue at the donor site where tissue was excised, can then heal over time.

The thickness of the graft may vary and can be dependent on the requirements, e.g. in a range of 1 mm to 2 mm, e.g. 1.0, 1.5 or 2.0 mm. Types of gingival grafts that are specifically of interest in the context of the present application are Free Gangival Grafts (FGG), and/or Connective Tissue Grafts (CTG). Particularly, an FGG, comprised of epithelial and lamina propria tissue, can be used to heal or restore gum tissue around teeth or implants. The FGG may furthermore be de-epithelialized, thus obtaining a CTG.

It is known in the art to use such conventional blade (e.g. no. 15-15c), or an alternative, such as a Paquette knife or a mucotome. Particularly, obtaining a graft of uniform thickness may pose challenges, especially when the patient is not edentulous. In a common approach, the FGG is freehandedly procured from the hard palate by the use of a scalpel, e.g. with a blade no. 15 or 15c. This can be a difficult technical procedure to perform. The natural curvature of the palate does not correspond well with the typical straight shape of a stiff blade. By repeated incisions to release the graft (epithelium and split thickness lamina propria) from the underlying connective tissue (submucosa), a ragged surface may be created on the underside of the graft and the exposed raw surface of the donor site. This may disadvantageously prolong the healing time of both the receptor and the donor site of the graft, and can increase the risk of post-operative complications. The thickness and width are also not tightly constrained, such that obtaining the desired dimensions requires substantial skill and experience. Various bowed blade designs for palatal surgery are also known, such as a Paquette blade or a Kewensky Modified Paquette blade.

An electrical mucotome can also be used, which uses a micro-motor system, for example with a quick coupling (e.g. an E-type coupling). However, this type of device can be particularly expensive, may be difficult to handle, and does not provide much flexibility in use. The mucotome may be adapted to cut a slice of fixed thickness and width, e.g. 0.5 mm by 6 mm, such that the potential usage over a range of applications is rather limited, even though it can have the advantage of being able to provide a clean cut of uniform thickness and width.

However, a need exists in the art for an instrument that allows the easy and effective extraction of a graft with even width and thickness, and smooth cut surface, in a time and cost-efficient manner. The use of reusable instruments and/or motor-driven equipment may come at a risk of contamination, and, moreover, motorized systems may carry a substantial cost.

The United States patent application no. US5330495A discloses a disposable gingival grafting knife with a narrow one-edged blade. The blade is embedded into the end of a sterilizable handle, and is formed to the desired shape of the graft to define a preselected width and thickness. Also, a detachable tissue graft receptacle is provided to the back of the knife near the blade, to catch and hold the harvested graft.

### Summary of the invention

It is an object of embodiments of the present invention to provide in means, e.g. surgical instruments and disposable surgical cutting blades, for easily and effectively extracting tissue material, e.g. for a graft, e.g. a gingival graft (and/or, more specifically, a FGG and/or CTG), with even width and thickness and/or with a smooth cut surface.

It is an advantage of embodiments of the present invention that tissue can be extracted in a time-efficient manner, e.g. by allowing a high-quality graft extraction to be performed in a limited time.

It is an advantage of embodiments of the present invention that an instrument is provided that is easy to use.

It is an advantage of embodiments of the present invention that tissue can be extracted in a cost-efficient manner, e.g. by using an inexpensive knife. Also, by combining a reusable surgical instrument with disposable blades, a cost-effective solution is provided. Furthemore, manufacturing and design costs can be advantageously low. For example, a manufacturing process of conventional no. 15 blades can be easily modified for the disposable blade in accordance with embodiments.

It is an advantage of embodiments of the present invention that a safe extraction of graft material can be achieved, e.g. carrying a low risk of cross-contamination.

It is an advantage of embodiments of the present invention that a good healing capacity at a graft receptor and donor site can be achieved, e.g. by providing a clean-cut graft surface.

It is an advantage of embodiments of the present invention that a high-quality harvesting and de-epithelization for a CTC graft can be easily and efficiently achieved.

It is an advantage of embodiments of the present invention that the usage and performance of embodiments provided by the invention is predictable, e.g. that a graft of desired width and/or thickness can be obtained.

It is an advantage of embodiments of the present invention that a selectable uniform FGG graft thickness can be achieved, e.g. in the range of 0.5 to 2.5 mm (e.g. 0.5, 1.0, 2.0 or 2.5mm). For example, different intermediate pieces can be used to achieve the desired thickness. By only relying on two intermediate elements (without limitation thereto), a graft extraction and/or de-epithelialization can be achieved for any of three (without limitation) different graft thickness. De-epithelialization (e.g. 0.5 mm) and three different graft thicknesses (1.0 mm, 1.5 mm and 2.0 mm) can be achieved with substantially the same tool.

It is an advantage of embodiments of the present invention that a graft slice can be harvested without repeated cuts, e.g. in a single one-slide motion (e.g. while including rotational motion components to bring the blade to the desired depth and/or to exit the blade from the tissue after executing the cut, as explained in further detail in the detailed description hereinbelow).

It is an advantage of embodiments of the present invention that a handle geometry is provided that is easy to use. For example, the angulation of the blade may improve handling, particularly in view of its use in the mouth cavity. Furthermore, the handle design in accordance with embodiments allows the graft to be viewed directly by the operator, i.e. direct vision on the graft is possible. A specifically adapted handle geometry can allow tissue to be cut at the correct angle, while getting around nearby teeth.

A disposable surgical blade, a sterilizable surgical blade holder, a sterilizable surgical knife handle, and/or a kit-of-parts comprising the aforementioned devices, in accordance with embodiments of the present invention, achieves the above objective.

In a first aspect, the present invention relates to a disposable surgical blade for harvesting tissue material. The blade comprises a U-shaped plate, in which the U-shaped plate comprises a (e.g. substantially flat) base part and two lateral parts that are connected by two corresponding joint regions to the flat base part. The base part has a sharpened frontal edge that continuously extends between the two joint regions so as to span the entire width of the base part. Each lateral part has a sharpened frontal edge that extends over at least a section of a frontal edge of the lateral part, such that the sharpened frontal edge of each lateral part abuts against and/or continuously joins with the sharpened frontal edge of the base part.

The base part may be substantially flat, e.g. flat, e.g. have a curvature radius of at least ten times the width of the base part, e.g. at least 50 times the width of the base part, or, alternatively, may deviate less than one tenth (e.g. less than one 50^{th}) of the width dimension from a flat plane (a virtual, flat reference plane anchored by the joint regions) in the normal direction to the plane (i.e. perpendicular to the plane). However, embodiments are not necessarily limited thereto (e.g. to a perfectly flat plane or a practical approximation thereof).

In other embodiments, the base part may be slightly curved, e.g. may have a spherical, cylindrical or parabolic shape (without limitation thereto), for example bulging outward, away from the side of the reference plane where the lateral parts are positioned, e.g. so as to form a copula or barrel shape. For example, the lateral parts may merge (i.e. continuously extend) in a smooth, but generally still U-shaped, curve into the (generally central bottom of) base part. Such design might advantageously allow more flexibility in cutting through tissue, e.g. when a straight (i.e. linear) path would be less optimal. For example, the operation of harvesting tissue may be considered to comprise, in a rough approximation, a scooping action, that can follow a not necessarily straight path as deemed optimal by the operating surgeon. Notwithstanding potential advantages of a (e.g. slightly) curved base part, it will be understood that a flat base part may also provide good results, particularly if a high degree of thickness uniformity, sharp and well-defined cut edges and/or a lineal shape of the extracted sample (or of the extraction lesion at the donor site) is considered to be particularly desirable.

A disposable surgical blade in accordance with embodiments of the present invention may be adapted for harvesting a gingival graft, e.g. a Free Gingival Graft and/or a Connective Tissue Graft. However, other surgical applications are not necessarily excluded. In an embodiment, the disposable surgical blade in accordance with embodiments may be adapted for harvesting a strip graft, e.g. having a with of only 0.5 mm to 4 mm, e.g. about 1 mm to about 2 mm.

In a disposable surgical blade in accordance with embodiments of the present invention, each lateral part may comprise a fixation element, e.g. a protrusion, opening, groove and/or slot, adapted to, e.g. shaped and configured to, cooperate with a corresponding (e.g. complementary) fixation element of a sterilizable surgical blade holder, e.g. in accordance with embodiments of the second aspect of the present invention, to (rigidly) mount the disposable surgical blade in or on the blade holder, e.g. so as to hold the blade in the blade holder in a fully constrained relative position and orientation with respect to the holder when mounted.

In a disposable surgical blade in accordance with embodiments of the present invention, the U-shaped plate may be of a thin stainless steel plate.

In a disposable surgical blade in accordance with embodiments of the present invention, the U-shaped plate may be of a thin plate of a metal or metal alloy that is suitable for surgical cutting tools.

In a disposable surgical blade in accordance with embodiments of the present invention, the joint regions may form right-angled corners, e.g. joining the lateral part and the base part at an angle in the range of 35° to 55°, preferably in the range of 40° to 50°, even more preferred in the range of 43° to 47°, e.g. 45° or 45.0°.

In a disposable surgical blade in accordance with embodiments of the present invention, the joint regions may form a curved transition between the lateral part and the base part with a predetermined curvature radius and/or may define (e.g. well-defined) angular corners between the lateral part and the base part.

In a disposable surgical blade in accordance with embodiments of the present invention, the width of the sharpened frontal edge of the (e.g. flat) base part may be in the range of 0.25 to 20 mm, e.g. 0.5 to 18 mm, e.g. 4 to 15 mm, e.g. in the range of 6 to 10 mm, e.g. about 8 mm, e.g. may be 8.0 mm.

In a disposable surgical blade in accordance with embodiments of the present invention, the sharpened frontal edge of each lateral part may be limited to a length in the range of 1 mm to 5 mm, preferably in the range of 3 to 5 mm, e.g. in the range of 3.5 mm to 4.5 mm, e.g. about 4 mm, e.g. 4.0 mm.

In a second aspect, the present invention relates to a sterilizable surgical blade holder for holding a disposable surgical blade, e.g. in accordance with embodiments of the first aspect of the present invention. The blade holder comprises a body and a fixation element on each lateral side of the body so as to hold the disposable surgical blade in a fixed position and orientation with respect to the body, when the blade is mounted, and such that the disposable surgical blade can be removed from the body after use. The fixation element is adapted to position the sharpened frontal edge of the base part of the disposable surgical blade parallel to, and at a predetermined distance to, a nearest surface of the body, such that a gap is formed between the body and the base part for receiving a slice of tissue of a uniform thickness corresponding to said predetermined distance, in use of the sterilizable surgical blade holder with the mounted disposable blade.

In a sterilizable surgical blade holder in accordance with embodiments of the present invention, the body may have a substantially cubic or cuboid shape, e.g. a rectangular cuboid shape, in which the body has rounded corners.

In a sterilizable surgical blade holder in accordance with embodiments of the present invention, the fixation element, e.g. comprising a protrusion, opening, groove, slot and/or resilient element, may be adapted to, e.g. shaped and configured to, cooperate with a corresponding (e.g. complementary) fixation element, e.g. a protrusion, opening, groove and/or slot, of the disposable surgical blade to (rigidly) mount the disposable surgical blade in or on the blade holder, e.g. so as to hold the blade in the blade holder in a fully constrained relative position and orientation with respect to the holder when mounted.

In a sterilizable surgical blade holder in accordance with embodiments of the present invention, said predetermined distance of the sharpened frontal edge of the (e.g. flat) base part of the disposable surgical blade, when mounted, to the nearest surface of the body may be in the range of 0.1 to 5 mm, e.g. equal to 0.5 mm, 1 mm, 1.5 mm, 2.0 mm, 2.5 mm and/or 3.0 mm.

In a sterilizable surgical blade holder in accordance with embodiments of the present invention, the fixation element may have a symmetry so that the disposable surgical blade can be mounted in two different, mutually opposite, orientations with respect to the body. The fixation element may be positioned excentrically so as to mount the frontal edge of the (e.g. flat) base part of the disposable surgical blade at a different distance (cf. the predetermined distance) to the nearest surface of body in the different orientations.

The invention may further relate to a set of at least two different sterilizable surgical blade holders in accordance with embodiments of the second aspect of the present invention, that may be shaped and configured substantially identically, except for a different position of the fixation elements, such that the predetermined distance, or the predetermined distances corresponding to the different orientations, differ between the at least two blade holders.

In a sterilizable surgical blade holder set in accordance with embodiments of the present invention, the predetermined distance may be equal to, for a first holder of the set of holders, 1 mm, in a first orientation of the different orientations, and 2 mm, in a second orientation of the orientations. In a sterilizable surgical blade holder set in accordance with embodiments of the present invention, the predetermined distance may be equal to, for a second holder of the set of holders, 0.5 mm, in a first orientation of said orientations, and 2.5 mm, in a second orientation of said orientations.

A sterilizable surgical blade holder in accordance with embodiments of the present invention may comprise an axial element adapted to removably mount the blade holder with a fastener to a sterilizable surgical knife handle so as to provide a degree of rotational freedom to the blade holder with respect to the handle around an axis of the axial element when the blade holder is mounted to the handle.

In a sterilizable surgical blade holder in accordance with embodiments of the present invention, the fixation elements on the opposite lateral sides of the body may be oriented at an angle in the range of 0.5° to 20°, e.g. in the range of 1 ° to 10°, e.g. in the range of 1 ° to 5°, around a lateral axis connecting the centers of said fixation elements with respect to the surface of the body nearest to the blade when mounted, such that that blade, when mounted, is at a slight angle. Additionally or alternatively, at least one face of the body may be slightly inclined (e.g. such that the shape of the body is only approximately cuboid, e.g. slightly assymetrical and/or trapezoid in shape). Thus, the same effect may be achieved as by orienting the fixation elements at a small angle, e.g. in which the suface of the body nearest to the blade slightly increases in distance to the blade so as to form a gap between the blade and the body that fractionally widens away from the cutting edge.

In a third aspect, the present invention relates to a sterilizable surgical knife handle for rotatably holding the sterilizable surgical blade holder. The sterilizable surgical knife handle comprises a grip, a receptacle adapted to hold the sterilizable surgical blade holder in a rotable orientation, an elongated body connecting the grip to the receptacle, and a fastener adapted to removably mount the sterilizable surgical blade holder to the surgical knife handle so as to provide a degree of rotational freedom around an axis to the blade holder with respect to the surgical knife handle when mounted, and adapted to releasably fix, e.g. lock, said degree of rotational freedom at a selectable angle.

In a sterilizable surgical knife handle in accordance with embodiments of the present invention, the elongated body may be curved and/or bent so as to position the receptacle away from an axis of the grip, in which the curved and/or bent elongated body defines an angle between the receptacle and the grip in the range of 15° to 45°, preferably in the range of 20° to 40°, even more preferred in the range of 25° to 35°, e.g. about 30°.

In a fourth aspect, the present invention relates to a kit-of-parts comprising a disposable surgical blade (e.g. in accordance with embodiments of the first aspect of the present invention) for harvesting tissue material, in which the disposable surgical blade comprises a U-shaped plate. The kit-of-part comprises further a sterilizable surgical blade holder (e.g. in accordance with embodiments of the second aspect of the present invention) for holding the disposable surgical blade, and a sterilizable surgical knife handle (e.g. in accordance with embodiments of the third aspect of the present invention) for rotatably holding the sterilizable surgical blade holder.

The U-shaped plate comprises a (e.g. substantially flat) base part and two lateral parts that are connected by two corresponding joint regions to the flat base part. The base part has a sharpened frontal edge that continuously extends between the two joint regions so as to span the entire width of the base part. Each lateral part has a sharpened frontal edge that extends over at least a section of a frontal edge of the lateral part, such that the sharpened frontal edge of each lateral part abuts against and/or continuously joins with the sharpened frontal edge of the base part.

The sterilizable surgical blade holder comprises a body, a fixation element on each lateral side of said body so as to hold the disposable surgical blade in a fixed position and orientation with respect to the body when mounted and such that the disposable surgical blade can be removed from the body after use. The fixation element is adapted to position the sharpened frontal edge of the base part of the disposable surgical blade parallel to, and at a predetermined distance to, a nearest surface of the body, such that a gap is formed between the body and the base part for receiving a slice of tissue of a uniform thickness corresponding to said predetermined distance, in use of the assembled kit-of-parts.

The sterilizable surgical knife handle comprises a grip, a receptacle adapted to hold the sterilizable surgical blade holder in a rotable orientation, an elongated body connecting the grip to the receptacle, and a fastener adapted to removably mount the sterilizable surgical blade holder to the surgical knife handle so as to provide a degree of rotational freedom around an axis to the blade holder with respect to the surgical knife handle when mounted, and adapted to releasably fix said degree of rotational freedom at a selectable angle.

The independent and dependent claims describe specific and preferred features of the invention. Features of the dependent claims can be combined with features of the independent claims and with features of other dependent claims as deemed appropriate, and not necessarily only as explicitly stated in the claims.

### Short description of the drawings

FIG 1 shows a disposable surgical blade for harvesting tissue material, e.g. a tissue graft, such as a gingival graft, in accordance with embodiments of the present invention.
FIG 2 explains a design of a disposable surgical blade in accordance with embodiments as based on the merging of two conventional no. 15 blades, to illustrate embodiments of the present invention.
FIG 3 shows a lateral view of an illustrative sterilizable surgical blade holder in accordance with embodiments of the present invention.
FIG 4 shows an illustrative sterilizable surgical blade holder in accordance with embodiments of the present invention, onto which the disposable surgical blade can be mounted in two different orientations (left-hand drawing vs. righthand drawing).
FIG 5 shows an illustrative sterilizable surgical knife handle in accordance with embodiments of the present invention for rotatably holding a blade holder in accordance with embodiments of the present invention, and with a mounted blade in accordance with embodiments of the present invention.
FIG 6 shows an illustrative sterilizable surgical knife handle in accordance with embodiments of the present invention for rotatably holding a blade holder in accordance with embodiments of the present invention, and with a mounted blade in accordance with embodiments of the present invention.
FIG 7 schematically illustrates the use of a rotatable arrangement of the holder in accordance with embodiments of the present invention with respect to the knife handle in accordance with embodiments of the present invention for harvesting tissue from the palate, regardless of the orientation of the donor site.
FIG 8 shows two different harvesting sites from where a graft sample can be collected from the palate, to illustrate a possible use of embodiments of the present invention.
FIG 9 illustrates a technique for harvesting a tissue sample in a single, swiping motion (assisted by rotational motion components) by use of embodiments of the present invention.

The drawings are schematic and not limiting. Elements in the drawings are not necessarily represented on scale. The present invention is not necessarily limited to the specific embodiments of the present invention as shown in the drawings.

### Detailed description of embodiments of the invention

Notwithstanding the exemplary embodiments described hereinbelow, is the present invention only limited by the attached claims. The attached claims are hereby explicitly incorporated in this detailed description, in which each claim, and each combination of claims as allowed for by the dependency structure defined by the claims, forms a separate embodiment of the present invention.

The word "comprise," as used in the claims, is not limited to the features, elements or steps as described thereafter, and does not exclude additional features, elements or steps. This therefore specifies the presence of the mentioned features without excluding a further presence or addition of one or more features.

In this detailed description, various specific details are presented. Embodiments of the present invention can be carried out without these specific details. Furthermore, well-known features, elements and/or steps are not necessarily described in detail for the sake of clarity and conciseness of the present disclosure.

In a first aspect, the present invention relates to a disposable surgical blade for harvesting tissue material, e.g. a tissue graft, such as a gingival graft. The surgical blade comprises a U-shaped plate, that comprises a substantially (e.g. flat) base part and two lateral parts connected, by two corresponding joint regions, to the base part. The base part has a sharpened frontal edge continuously extending between the two joint regions. Each lateral part has a sharpened edge that extends over at least a section of a frontal edge of the lateral part so as to abut against and/or continuously join with the sharpened edge of the base part.

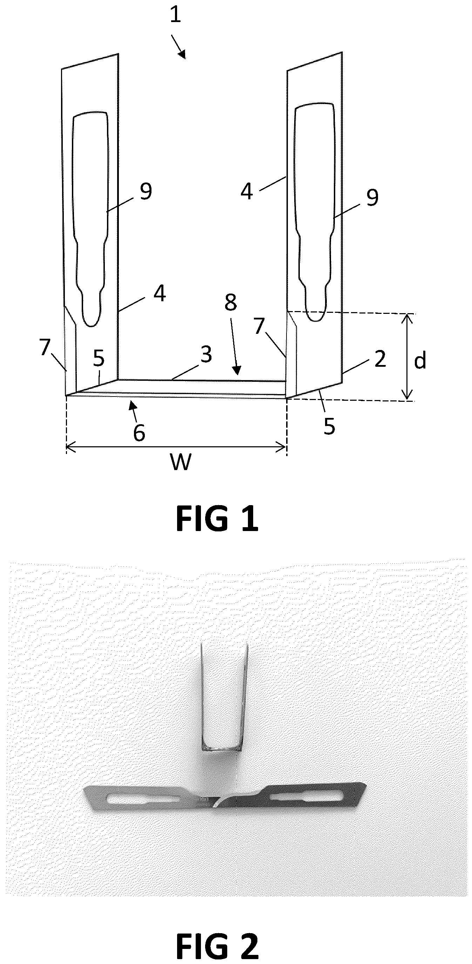

Referring to FIG 1, an illustrative disposable surgical blade 1 in accordance with embodiments of the present invention is shown. The blade 1 comprises, e.g. consists or is composed of, a plate 2 having a U-shaped profile. For example, the plate (e.g. substantially the entire blade) may be made from a thin metal plate, e.g. a stainless steel plate, e.g. made of a high-grade stainless steel, a steel specifically for knifes and bladed tools, a surgical grade steel, and/or generally any suitable metal or other suitable material, e.g. a metal alloy or combinations thereof, e.g. a carbine (e.g. tungsten carbide), iron, tungsten, molybdenum, vanadium, chromium, cobalt, aluminium and alloys or combinations thereof.

The U-shaped plate comprises a base part 3, which may be substantially flat, e.g. within practical and/or economical limitations and/or within manufacturing tolerances. Alternatively, the base part may be slightly curved, e.g. bulging outward away from the side where the lateral parts 4 are located. The shape of such slightly curved base part may be spherical, ovoid, cylindrical, parabolic, and/or generally any suitable (e.g. convex) shape.

The plate 2 further comprises two lateral parts 4, which are connected to the base part through two corresponding joint regions 5. The joint regions may be right-angled corners, e.g. joining the lateral part and the base part at an angle in the range of 35° to 55°, preferably in the range of 40° to 50°, even more preferred in the range of 43° to 47°, e.g. 45° or 45.0°. This corner may be formed by joining two separate pieces of (e.g. metal) plate, e.g. by a welding, glueing or, generally, any suitable technique, but may also be integrally formed from a single sheet, e.g. by plying the material to obtain a right, or substantially right, angle. The joint region may also comprise an arched transition, e.g. obtainable by folding a sheet of material into a right angle, such that a predetermined curvature radius remains in the corner.

Otherwise said, the joint may be a clearly defined angular corner or a good approximation thereof (within technical capabilities and/or in view of manufacturing considerations), or may be (e.g. intentionally) curved along a predetermined curvature radius (in the frontal viewing plane of the U profile). For example, it may be advantageous to provide some curvature (e.g. chosen to be more than strictly technically necessary), to obtain grafts, using the blade, with a naturally decreasing height slope around (and close to) its edge and/or a smoothed cut at the lateral sides. For example, this might improve healing at the donor and/or receptor site and/or reduce a risk of infection (e.g. sharp excision corners potentially providing a niche pocket for harmful bacteria and/or being poorly reachable by disinfectants, medication and/or saliva). A rounded corner may also improve handling safety of the blade, and/or may avoid undesirable strong stresses in the blade material (e.g. metal), and/or, obviously, may be more practical to manufacture when obtained by plying a plate. However, a well-defined angular corner may equally be considered to have advantages, and is therefore not necessarily excluded, e.g. to maximize the uniformity of the the graft thickness over its entire area (or a good/best approximation thereof).

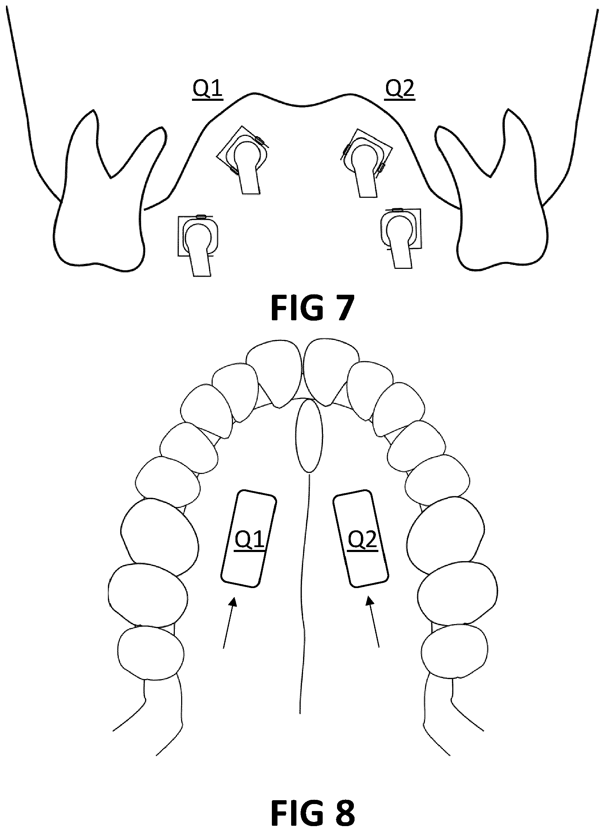

The base part 3 has a sharpened frontal edge 6, which continuously extends between the two joint regions 5, i.e. spans the entire width W of the base part 3, e.g. along an (one of the two) edge(s) thereof that is perpedicular to both joints. The width W of the sharpened frontal edge 4, and thus of the base part, may be in the range of 0.25 mm to 20 mm, e.g. 0.5 mm to 4 mm (e.g. 1 mm to 2 mm), and/or in the range of 4 to 15 mm, e.g. in the range of 6 to 10 mm, e.g. about 8 mm, e.g. may be 8.0 mm. A width W of 8.0 mm (or, alternatively, a value close thereto) may be a particularly advantageous choice, since this corresponds to a most commonly used gingival graft width (e.g. more or less oriented in the lateral direction when considering the donor site in the mouth cavity, as is discussed further hereinbelow in relation to FIG 8).

Each lateral part 4 furthermore has a sharpened frontal edge 7 that extends over at least a section of a frontal edge of the lateral part (the frontal edge which continues into the frontal edge of the base part), so that the sharpened edge of the lateral part abuts against and/or continuously joins with (merges into) the sharpened frontal edge 6 of the base part. Thus, a continuous (or substantially continuous) cutting edge is formed by the sharpened frontal edge 6 and the two sharpened lateral frontal edges 7, i.e. to form a U-shaped (e.g. with substantially flat bottom) excision tool.

The sharpened edges 6,7 may be formed by tapering the material of the plate along the relevant sections of the plate's frontal edge into a sharp and clean edge suitable for surgical cutting procedures (e.g. tissue harvesting, e.g. for obtaining a graft), for example by suitable metalworking and/or other material fabrication techniques.

Thus, the bottom portion (base part 3, entirely, and at least a section of both the lateral parts 4) of the blade plate 2 is sharpened at at least one edge (referred to as the frontal edge, without implied limitations) to create a blade for a cutting instrument, particularly one suitable for graft harvesting, e.g. gingival graft harvesting. Optionally, the opposite edge (rear edge 8, i.e. of the base part and abutting sections of the lateral parts) may be sharpenened to provide a blade that can be used at both sides.

Even though the lateral parts 4 may be sharpened over substantially the entire length of the the frontal edge, it may be preferable to limit the sharpened edge(s) 7 to a portion thereof. Thus the sharpened edge 7 may have a length d, e.g. in the range of 1 mm to 5 mm, preferably in the range of 3 to 5 mm, e.g. in the range of 3.5 mm to 4.5 mm, e.g. about 4 mm, e.g. 4.0 mm. Preferably, the length d corresponds to at least the maximum thickness of a tissue layer (e.g. graft to be harvested) that the blade is adapted to harvest. For example, 4 mm (or a value close thereto) is a particularly advantageous choice for the length d, since it provides ample cutting edge length to harvest graft material up to 2.5 mm thick (as will be explained more in detail below).

Furthermore, each lateral part 4 may comprise a fixation element 9, e.g. a protrusion, opening, groove and/or slot, to mount the disposable surgical blade 1 in or on a blade holder. For example, a slot 9 shaped to mount the blade into/onto the blade holder by corresponding fixation elements 19, i.e. via both lateral sides, may be used. Various variations of blade fixation means and corresponding designs of protrusions, openings, grooves and/or slots in the blade are known in the art. For example, a fixation element, e.g. a resilient element, may be inserted into the slot to hold it in a fixed position after pushing it through a cycle of elastic deformation into a new potential energy minimum where the slot can only be removed from the fixation element by overcoming a potential energy barrier by a (opposite) extraction force. Examples of slots and resilient elements, e.g. to provide a slide-and-click, turn-and-click, click-on-click-off or other similar fastening procedure, are well-known in the field, and therefore not discussed in further detail.

The fixation element 9 (e.g. protrusion(s), opening(s), groove(s) and/or slot(s)) may be shaped such as to hold the blade in a fully constrained (for translations and rotations) relative position (at at least two points, i.e. both lateral sides) with respect to the holder when mounted, at least until a force is applied (in the appropriate direction) to overcome the potential energy barrier of the engaged fixation elements. For example, as shown in FIG 1, the slot may be wider at the side distal to the base part 3 than on the side proximal to the base part, such that it can be slided over and pushed (clicked) onto the fixation element and later removed by pulling it back (clicking it off).

The blade 1 may be particularly suitable for, i.e. specifically adapted for, harvesting a tissue layer, e.g. a gingival graft, e.g. a Free Gingival Graft and/or Connective Tissue Graft.

The illustrative blade may be understood as a merging of a conventional no. 15 blade with its mirror image, after bending the no. 15 blade in a right angle. This principle is shown in FIG 2, for illustrative purposes. The slot designs 9, overall dimensions, edge properties, and/or other characteristics of the blade may thus correspond to those obtained by such mirror combination of conventional no. 15 blades known in the art (obviously, after excluding the overlap region where the blades are joined in this illustrative example). However, it will be understood that, for practical use, the blade edge of the base part (e.g. the entire blade plate) may be integrally formed to obtain a single sharp cutting edge, unlike an overlap and juxtaposition of the edges obtained by merely combining two folded conventional blades.

For example, the slot design of a conventional no. 15 blade may be used in the blade 1, even though alternatives as known in the art are certainly not considered to be excluded in embodiments. The illustrative conventional slot has a length of 18 mm, and is shaped to stepwise reduce in width (direction perpedicular to the length), as shown.

In a second aspect, the present invention relates to a sterilizable surgical blade holder for holding the disposable blade in accordance with embodiments of the first aspect of the present invention. In a third aspect, the present invention relates to a sterilizable surgical knife handle for rotatably holding the blade holder, comprising a fastener adapted to mount and to remove the blade holder to the handle so as to provide a degree of rotational freedom to the blade holder with respect to the handle when mounted. The fastener is furthermore adapted to reversibly fix the blade holder at a selectable angle.

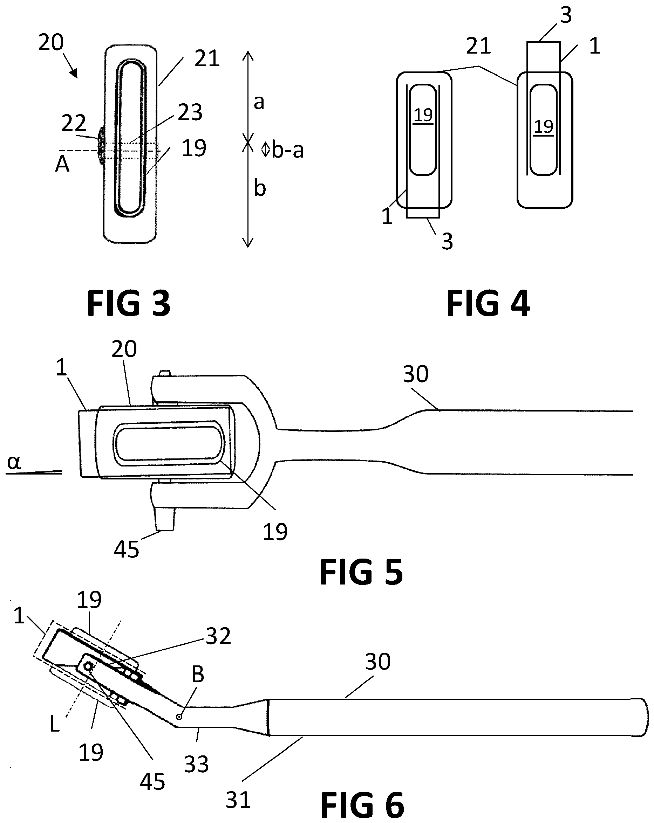

FIG 3 shows a lateral view of an illustrative sterilizable surgical blade holder 20 in accordance with embodiments of the present invention.

The blade holder 20 comprises a body 21, e.g. made of a sterilizable material (e.g. by chemoclave and/or autoclave), such as a suitable metal or plastic (e.g. stainless steel, titanium, a temperature-resistant polymer, Plexi glass, glass, ...). The body may be solid or hollow. The body may have a substantially cubic or cuboid shape, e.g. a rectangular cuboid shape. The body may have rounded corners, to aid in sliding over tissue while cutting, with an attached blade, a layer of tissue underneath a surface of the body contacting, e.g. pressing against, the tissue's upper surface.

On each lateral side of the body (shown in the front view of FIG 3), a fixation element 19, e.g. a protrusion, opening, groove, slot and/or resilient element, is provided that is adapted to cooperate with the corresponding fixation means 9 of the blade 1 so as to hold and fix the blade, in a removable configuration (such as to allow deattachment). In this example, the blade can be removably fixed onto the blade holder 20 by clicking the slots 9 (cf. FIG 1) over the protrusions 19 on both lateral sides of the body. Once an initial required force is applied, the blade moves into its fixed position for use, where it is trapped by a potential force barrier (e.g. provided by resilience of the protrusion or a part thereof). The blade can, after use, be removed, by an opposite force to overcome this barrier. It will be noted that this is only an illustrative approach for deattachable fixation, and other approaches of cooperating fixation elements 9 and 19, as known in the art, can be easily conceived in view of the state of the art.

The fixation element (19) is adapted to position the frontal cutting edge of the base part of the blade, when mounted, parallel to, and at a predetermined distance to, a nearest surface of the body 21. Thus, a (e.g. air) gap is formed between the body and the base part of the mounted blade to receive a slice of tissue of a uniform thickness that corresponds to this predetermined distance.

Preferably, the fixation element 19 has a symmetry so that the blade can be mounted in two different orientations, and provided excentrically, illustrated by the different distances a and b between the rim of the body and the center of the fixation element, such that in these mounting configurations the base part of the blade (particularly, the frontal cutting edge) is mounted at a different distance from the body. FIG 4 illustrates this principle by showing two different configurations (left and right side drawing) of the mounted blade 1. It will be noted that, due to the excentric position of the fixation element 19, the base part 3 of the blade will be at a different distance from the holder when comparing these onfigurations.

This also allows to provide a set of at least two of the holders which have a different position of the fixation element on the lateral side of the body, such that a desired distance of the blade's base part to the body of the holder can be selected by choosing the corresponding holder alternative. By combining this with the symmetrical design and excentric position of the fixation element, described hereinabove (cf. FIG 4), the number of selectable (supported) positions of the base part, relative to the holder's body, can be twice the number of alternative holders in the set.

For example, a first holder may provide a distance adapted to extract a 1 mm thick tissue layer (e.g. for a FGG) in a first configuration (a first orientation of the blade), and a 2 mm thick tissue layer (e.g. for a FGG, or a CTG after extra-oral deepithelialization) in a second configuration (a second orientation of the blade). In other words, the distance between the mounted blade's base part and the holder body may be substantially 1 mm in the first configuration, and substantially 2 mm in the second configuration.

A second holder may provide a distance adapted to harvest a 0.5 mm thick tissue layer in a first configuration (a first orientation of the blade), and a 2.5 mm thick tissue layer in a second configuration (a second orientation of the blade). In other words, the distance between the mounted blade's base part and the holder body may be substantially 0.5 mm in the first configuration, and substantially 2.5 mm in the second configuration. The second configuration can be used to cut a 2.5 mm thick tissue layer from the palate, in a first cutting slide. Then, a second slide over the donor site with the former configuration of the blade (having flipped the blade), deepithelializes the 2.5 mm thick tissue sample to obtain a 2 mm Connective Tissue Graft. Thus, advantageously, a CTC can be obtained with only two sliding cuts while requiring little manipulation of the equipment or additional instruments.

In an embodiment, the fixation elements 19 on the opposite lateral sides of the holder's body, for engaging with the corresponding fixation elements 9 of the blade, may be slightly oriented at a small angle *α* (i.e. both in the same angle and direction) in the range of 0.5° to 20°, e.g. in the range of 1° to 10°, e.g. in the range of 1° to 5°, around a lateral axis L (connecting the centers of both fixation elements) with respect to a base surface of the body that is proximal to the blade when mounted, such that that blade, when mounted, is at a slight angle. The orientation illustrated in FIG 5 may be exaggerated for illustrative purposes. This allows the base part 3 of the blade to be at its closest distance to the holder's body at the cutting edge 6, such that a tissue sample, cut at the cutting edge and pressed against the holder's body (to esure a uniform and predetermined thickness), can easily slide over the blade, i.e. due to a less constricted space between the blade and the body away from the cutting edge.

Referring to FIG 5, an illustrative sterilizable surgical knife handle 30 for rotatably holding the blade holder, in accordance with embodiments, is schematically shown, with a sterilizable blade holder 20 in accordance with embodiments mounted thereto.

Similarly to the sterilizable blade holder 20, the sterilizable surgical knife handle 30 may be made of a sterilizable material (e.g. by chemoclave and/or autoclave), such as a suitable metal or plastic (e.g. stainless steel, titanium, a temperature-resistant polymer, Plexi glass, glass, ...).

The blade holder 20 may be adapted to be rotatably mounted on the handle 30, e.g. by the fastener 45, such that a rotational degree of freedom is provided between the blade holder and the handle. The fastener 45 may comprise a screw, a bolt, an axle and/or other suitable fixation means. Preferably, the fastener 45 is adapted to lock the rotation of the blade holder 20 with respect to the handle, e.g. by tightening the screw. For example, the holder may comprise a nut, threaded opening or similar means 22 for holding and fixing the fastener 45, e.g. a screw or bolt (and, thus, when tightened, fix the position of the holder). The fastener 45 may furthermore be adapted to allow the blade holder to be released from the handle, i.e. to removably (in other words, reversibly) mount the holder. In more general terms, the holder may comprise an axial element 23 adapted to cooperate with the fastener so as to allow rotation around an axis A of the axial element when the holder is mounted to (e.g. in, e.g. on) the handle, wherein the fastener and axial element are adapted to allow the removal of the holder from the handle (e.g. by removing the fastener), to allow a free rotation (which may be constrained to a predetermined angular range, or entirely free) of the holder around the axis, and to allow a selected angle around said axis to be (reversibly) fixed.

In the illustrated example, the fastener 45 is a screw or bolt, and the corresponding axial element 23 in an opening through the holder for receiving the screw or bolt. For example, the axial element 23 may comprise, e.g. in the illustrated examples, a through-hole through the body of the holder, through which the fastener (e.g. screw or bolt) can be inserted. The handle may comprise complementary through-holes through lateral sides of a prong (for, also, receiving the fastener), in between which the holder can be placed and held. A nut, or similar tightening device, may be comprised in (e.g. integrated in, fixed to) the holder, or may be provided (or relied upon) as a separate component, e.g. the screw or bolt and nut may be provided as independent loose parts.

The holder may freely rotate, when mounted to the handle, but can be fixed at a selected angle, e.g. by a nut 22 or similar fastening means adapted to cooperate with the fastener 45, e.g. by tightening the screw or bolt. However, the skilled person can consider many alternatives, without inventive effort, to provide a freedom of rotation around the axis A and/or to reversibly fix the rotation at a desired angular position. For example, instead, the handle my comprise axially aligned openings in a prong, wherein axially aligned protrusions on the holder can be placed to freely rotate around the axis. Axial fixation may then be provided by clamping one or two ends of the protrusions using a releasable clamping mechanism on the handle. However, it will be understood that the proposed example, as shown in the drawings, may be advantageously simple and effective.

An elongated body 33 of the handle, connecting a grip 31 to a receptacle 32 for the holder, may be curved or bent, so as to position the receptacle away from an axis of the handle. The angle between the receptacle and the grip, provided by said curved or bent elongated body, may be in the range of 15° to 45°, preferably in the range of 20° to 40°, even more preferred in the range of 25° to 35°, e.g. about 30°. In other words, the elongated body may be bent or curved in this angle. The positional displacement of the receptacle with respect to the axis of the grip may be particularly suitable for periodontal surgery, e.g. allowing easy access around the teeth in the mouth cavity.

The receptacle 32 is adapted to hold the holder in a rotable, but reversible fixable, position. For example, the receptacle may comprise a prong, in which openings are provided, in both arms of the prong, to receive the fastener 45, to provide a freedom of rotation to the holder around the axis A (which is e.g. perpendicular to the plane of the drawing in FIG 6, e.g. at the center of fastener 45). The predetermined angle provided between the receptacle and the grip, by the elongated body, may be around an axis (cf. axis B) that is substantially parallel to the axis A.

When a blade is mounted on the holder, which is in turn is mounted on the handle, the fixation elements 19 of the holder (on two lateral sides of the holder) may be connected by a (virtual) line (e.g. between the geometric centers of the fixation elements) that is perpendicular to the axis A. It will be noted that this allows the blade to be mounted without the lateral parts of the blade interfering with the means for rotatably mounting the holder to the handle.

The blade, holder and handle are intended to be used in combination, e.g. are cooperating parts to reduce the present invention to practice. However, it will be understood that each can be commercialized in its own right, i.e. can be sold as a separate product. In a fourth aspect, the present invention relates to a kit-of-parts, comprising the blade in accordance with embodiments, the holder in accordance with embodiments and the handle in accordance with embodiments.

The use and applications of embodiments in accordance with the present invention will now be discussed hereinbelow. Embodiments of the present invention are primarily intended for, or particularly suitable for, the harvesting of a tissue sample, e.g. a layer of tissue(s), e.g. a graft. However, other surgical applications are not necessarily excluded.

The invention advantageously allows to cut tissue underneath the sample to be collected and, simultaneously, at the sides (defining the thickness of the sample). The embodiments may be particularly suitable for harvesting a tissue sample of substantially uniform thickness, e.g. in the range of 0.1 to 10 mm, e.g. in the range of 0.5 mm to 4 mm, e.g. in the range of 0.5 mm to 2.5 mm. Embodiments may be adapted for the harvesting of a tissue sample of substantially uniform thickness. Likewise, a predetermined and uniform width of the sample can be obtained, cf. corresponding to the length of the cutting edge of the base part of the blade.

The blades may be disposable, so as to avoid contamination and damage of the blade (dulling of the edges). The holder(s) and handle may be reusable, e.g. easily sterilizable.

Whereas a detachable blade holder and handle are described hereinabove, it is noted that embodiments in which the blade holder is integrated into the handle (preferably in a rotable and lockable arrangement) are not necessarily excluded either. Attention is drawn, however, to the specific advantages of embodiments in which the blade holder is detachable, e.g. such that a different holder can be attached in view of the particular requirements of each individual use of the device.

Embodiments of the invention may be particularly suitable for harvesting a gingival graft, e.g. a free gingival graft (FGG) and/or connective tissue graft (e.g. such CTG may also be considered to be a more specific type of FGG). The thickness may be easily selectable from at least two different selectable thicknesses, e.g. by placing the blade on the holder in one of two, opposite, orientations, as discussed hereinabove. Likewise, even more selectable thicknesses may be supported, e.g. 2x2, by providing two different holders (each supporting two different thicknesses by reversing the blade) that can be easily mounted (however, not simultaneously) to the same handle.

The rotatable arrangement of the holder (and, thus, indirectly, also the blade) with respect to the handle, allows the instrument (obtained by the assembled kit-of-parts) to be used to easily harvest tissue regardless of the orientation of the donor site. For example, for extracting tissue from the palate, the angular position can be easily adjusted (and fixed) to accommodate for the variable curvature of the palate, as illustrated in FIG 7, i.e. the different orientation of the normal to the palate at different potential graft harvesting sites.

FIG 8 shows, for example, two different harvesting sites, Q1 and Q2, from where a graft sample can be collected from the palate. By the adjustable orientation of the holder, different curvature of the palate can be easily compensated. The procedure may generally be carried out by a swiping motion in (approximately) a posterioanterior direction (cf. the arrow indicators). Since 8 mm is the most common graft width, the width of the base part of the blade plate may substantially correspond to this dimension in accordance with at least some embodiments.

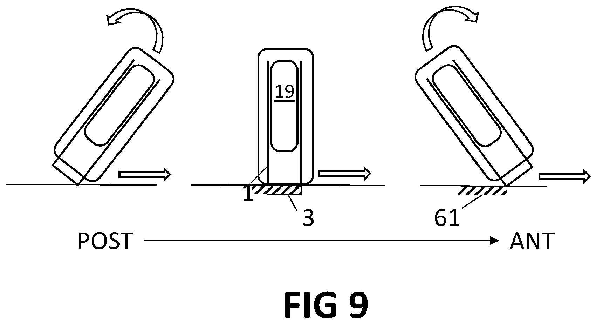

FIG 9 illustrates how a tissue sample 61 can be cut in substantially a single, swiping motion, in the posterior (POST) to anterior ANT direction (for the present illustrative application). First, the blade can be brought at depth in a rotational motion, the sample 61 can be cut from the tissue underneath (e.g. submucosa) by a translative, swiping motion, and, finally, the blade can be brought out by a second rotational motion. This results in a piece of tissue material, of uniform width and thickness, completely separated from the surrounding tissue in only a single, simple manipulation and by only a single surgical instrument.

However, it is to be noted that this procedure is only intended for illustrative purposes. Variations and/or alternative procedures and/or uses of devices in accordance with embodiments can be considered by the person skilled in the art. The scope of the present invention is limited, cf. the appended claims, to instruments and/or devices suitable for use in medical, e.g. periodontal, practice, and potentially related methods, such as for manufacturing such devices, but excludes any method for treatment of the human or animal body by surgery or therapy, at least in so far as is required by the applicable laws, e.g. in view of protection of the freedom to practice of the medical professions.

Furthermore, in accordance with at least some embodiments, the tissue sample thickness can be easily selected by installing the blade in one orientation or another orientation on the blade holder (cf. FIG 4), and even further thicknesses may be available by selecting an appropriate blade holder from a plurality of options. For example, a 1 mm or 2 mm FGT thickness can be directly obtained by using a single holder. Another blade holder may be used that allows a layer of 0.5 mm or 2.5 mm to be harvested. For example, in a first cutting slide, a 2.5 mm thick layer of tissue may be released from the palate, and in a second cutting slide (after reversal of the holder), the epithelium may be removed from the sampled tissue to obtain a 2 mm thick CTG.

Other features, or details of the features described hereinabove, of a blade, holder, handle and/or kit-of parts in accordance with embodiments of the present invention shall be clear in view of the description provided hereinabove relating to embodiments of the other aspects of the present invention.

## Claims

1. A kit-of-parts comprising:
- a disposable surgical blade (1) for harvesting tissue material, said blade comprising a U-shaped plate (2),
- a sterilizable surgical blade holder (20) for holding the disposable surgical blade (1), and
- a sterilizable surgical knife handle (30) for rotatably holding the sterilizable surgical blade holder (20),
wherein said U-shaped plate comprises a base part (3) and two lateral parts (4) that are connected by two corresponding joint regions (5) to the base part,
wherein said base part (3) has a sharpened frontal edge (6) that continuously extends between the two joint regions (5) so as to span the entire width (W) of the base part,
wherein each lateral part (4) has a sharpened frontal edge (7) that extends over at least a section of a frontal edge of the lateral part, such that the sharpened frontal edge (7) of each lateral part abuts against and/or continuously joins with the sharpened frontal edge (6) of the base part,
wherein said sterilizable surgical blade holder (20) comprises:
- a body (21),
- a fixation element (19) on each lateral side of said body so as to hold the disposable surgical blade (1) in a fixed position and orientation with respect to the body (21) when mounted and such that the disposable surgical blade (1) can be removed from the body after use,
wherein said fixation element (19) is adapted to position the sharpened frontal edge (7) of the base part (3) of the disposable surgical blade (1) parallel to, and at a predetermined distance to, a nearest surface of the body (21), such that a gap is formed between the body (21) and the base part (3) for receiving a slice of tissue of a uniform thickness corresponding to said predetermined distance, in use of the sterilizable surgical blade holder (20) with the mounted disposable blade (1),
wherein said sterilizable surgical knife handle (30) comprises:
- a grip (31),
- a receptacle (32) adapted to hold the sterilizable surgical blade holder (20) in a rotable orientation,
- an elongated body (33) connecting the grip (31) to the receptacle (32), and
- a fastener (45) adapted to removably mount the sterilizable surgical blade holder (20) to the surgical knife handle so as to provide a degree of rotational freedom around an axis (A) to the blade holder with respect to the surgical knife handle when mounted, and adapted to releasably fix said degree of rotational freedom at a selectable angle.

2. A disposable surgical blade (1) for harvesting tissue material, said blade comprising a U-shaped plate (2),
wherein said U-shaped plate comprises a base part (3) and two lateral parts (4) that are connected by two corresponding joint regions (5) to the base part,
wherein said base part (3) has a sharpened frontal edge (6) that continuously extends between the two joint regions (5) so as to span the entire width (W) of the base part,
wherein each lateral part (4) has a sharpened frontal edge (7) that extends over at least a section of a frontal edge of the lateral part, such that the sharpened frontal edge (7) of each lateral part abuts against and/or continuously joins with the sharpened frontal edge (6) of the base part.

3. The disposable surgical blade of claim 2, wherein said disposable surgical blade (1) is adapted for harvesting a gingival graft, a Free Gingival Graft and/or Connective Tissue Graft.

4. The disposable surgical blade of claim 2 or claim 3, wherein each lateral part (4) comprises a fixation element (9) adapted to cooperate with a corresponding fixation element (19) of a sterilizable surgical blade holder (20) to mount the disposable surgical blade (1) in or on the blade holder.

5. The disposable surgical blade of any of the claims 2 to 4, wherein said joint regions (5) form right-angled corners, and/or wherein said joint regions (5) form a curved transition between the lateral part (4) and the base part (3) with a predetermined curvature radius and/or define angular corners between the lateral parts (4) and the base part (3).

6. The disposable surgical blade of any of the claims 2 to 5, wherein said width (W) of the sharpened frontal edge (6) is in the range of 0.25 mm to 20 mm, in the range of 0.5 mm to 2.5 mm, or in the range of 4 to 15 mm, and/or wherein said sharpened frontal edge (7) of each lateral part is limited to a length (d) in the range of 1 mm to 5 mm.

7. A sterilizable surgical blade holder (20) for holding a disposable surgical blade (1), the blade holder comprising:
- a body (21),
- a fixation element (19) on each lateral side of said body so as to hold the disposable surgical blade (1) in a fixed position and orientation with respect to the body (21) when mounted and such that the disposable surgical blade (1) can be removed from the body after use,
wherein said fixation element (19) is adapted to position the sharpened frontal edge (7) of the base part (3) of the disposable surgical blade (1) parallel to, and at a predetermined distance to, a nearest surface of the body (21), such that a gap is formed between the body (21) and the base part (3) for receiving a slice of tissue of a uniform thickness corresponding to said predetermined distance, in use of the sterilizable surgical blade holder (20) with the mounted disposable blade (1).

8. The sterilizable surgical blade holder (20) of claim 7, wherein said body (21) has a substantially cubic or cuboid shape, and in which the body has rounded corners,
and/or wherein the sterilizable surgical blade holder (20) comprises an axial element (23) adapted to removably mount the sterilizable surgical blade holder with a fastener (45) to a sterilizable surgical knife handle (30) so as to provide a degree of rotational freedom to the blade holder with respect to the handle (30) around an axis (A) of the axial element when the blade holder is mounted to the handle.

9. The sterilizable surgical blade holder (20) of claim 7 or claim 8, wherein said fixation element (19) is adapted to cooperate with a corresponding fixation element (9) of said disposable surgical blade (1) to mount the disposable surgical blade (1) in or on the blade holder, and/or wherein said predetermined distance of the sharpened frontal edge (6) of the base part (3) of the disposable surgical blade (1), when mounted, to the nearest surface of the body (21) is in the range of 0.1 to 5 mm.

10. The sterilizable surgical blade holder (20) of any of the claims 7 to 9, wherein said fixation elements (19) on the opposite lateral sides of the body (21) are oriented at an angle in the range of 0.5° to 20° around a lateral axis connecting the centers of said fixation elements with respect to the surface of the body nearest to the blade when mounted, such that that blade, when mounted, is at a slight angle.

11. The sterilizable surgical blade holder (20) of any of the claims 7 to 10, wherein said fixation element (19) has a symmetry so that the disposable surgical blade (1) can be mounted in two different, mutually opposite, orientations with respect to the body (21), and wherein said fixation element (19) is positioned excentrically so as to mount the frontal edge (6) of the base part (3) of the disposable surgical blade (1) at a different distance to the nearest surface of body (21) in the different orientations.

12. A set of at least two different sterilizable surgical blade holders (20) is accordance with any of the claims 10 to 15, wherein said sterilizable surgical blade holders (20) are shaped and configured substantially identically, except for a different position of said fixation elements (19), such that said predetermined distance, or the predetermined distances corresponding to said different orientations in accordance with claim 13, differ between said at least two different sterilizable surgical blade holders (20).

13. The set of sterilizable surgical blade holders of claim 12, wherein said predetermined distance is equal to, for a first holder of said at least two different sterilizable surgical blade holders (20), 1.0 mm, in a first of said orientations, and 2.0 mm, in a second of said orientations, and, for a second holder of said at least two different sterilizable surgical blade holders, 0.5 mm, in a first of said orientations, and 2.5 mm, in a second of said orientations.

14. A sterilizable surgical knife handle (30) for rotatably holding a sterilizable surgical blade holder (20) in accordance with any of the claims 10 to 17, the sterilizable surgical knife handle (30) comprising:
- a grip (31),
- a receptacle (32) adapted to hold the sterilizable surgical blade holder (20) in a rotable orientation,
- an elongated body (33) connecting the grip (31) to the receptacle (32), and
- a fastener (45) adapted to removably mount the sterilizable surgical blade holder (20) to the surgical knife handle so as to provide a degree of rotational freedom around an axis (A) to the blade holder with respect to the surgical knife handle when mounted, and adapted to releasably fix said degree of rotational freedom at a selectable angle.

15. The sterilizable surgical knife handle (30) of claim 14, wherein said elongated body (32) is curved and/or bent so as to position the receptacle (32) away from an axis of the grip (31), wherein the curved and/or bent elongated body defines an angle between the receptacle and the grip in the range of 15° to 45°.
